# EUROPÄISCHE PATENTSCHRIFT

(11) **EP 0 464 045 B1**
(45) Veröffentlichungstag und Bekanntmachung des Hinweises auf die Patenterteilung: **15.12.1993**
(21) Anmeldenummer: 90904289.7
(22) Anmeldetag: 12.03.1990
(51) Int. Cl.: B01D 3/00, B01J 14/00, B01J 19/18, C07C 67/02, C07C 67/08, C07C 69/22, C07C 69/58, C07C 69/60, C07C 69/80

(54) **DISKONTINUIERLICHES VERFAHREN ZUM FÜHREN EINER HETEROGEN KATALYSIERTEN REAKTION UND ANLAGE ZUM HETEROGEN KATALYSIERTEN HERSTELLEN VON PRODUKTEN**
DISCONTINUOUS PROCESS FOR CONDUCTING A HETEROGENEOUSLY CATALYSED REACTION AND INSTALLATION FOR HETEROGENEOUSLY CATALYSED MANUFACTURE OF PRODUCTS
PROCEDE EN DISCONTINU PERMETTANT DE PRODUIRE UNE REACTION A CATALYSE HETEROGENE ET INSTALLATION POUR LA PRODUCTION DE PRODUITS PAR CATALYSE HETEROGENE

(30) Priorität: 20.03.1989 DE 3909128
(43) Veröffentlichungstag der Anmeldung: 08.01.1992
(73) Patentinhaber: Henkel Kommanditgesellschaft auf Aktien, 40191 Düsseldorf (DE)
(72) Erfinder: GUTSCHE, Bernhard, D-4010 Hilden (DE); JEROMIN, Lutz, D-4010 Hilden (DE); PEUKERT, Eberhard, D-4010 Hilden (DE); YÜKSEL, Levent, D-4000 Düsseldorf 13 (DE); ADRIAN, Kurt, D-5205 St. Augustin-Hangelar (DE); BOLLWEG, Heinz, D-5205 St. Augustin-Birlinghoven (DE)
(86) Internationale Anmeldenummer: EP9000398
(87) Internationale Veröffentlichungsnummer: WO9011114

(56) Entgegenhaltungen:
- EP-A- 178 669
- EP-A- 342 357
- FR-A- 2 185 613
- FR-A- 2 293 238

## Beschreibung

Die Erfindung betrifft ein diskontinuierliches Verfahren zum Führen einer heterogen katalysierten und bei erhöhter Temperatur ablaufenden Reaktion, bei der thermisch empfindliche Produkte entstehen, wobei zum Energieeintrag ein vom Reaktor unterschiedlicher Wärmetauscher und als Katalysator ein Festbettkatalysator eingesetzt wird und das Reaktionsgemisch kontinuierlich im Kreislauf nacheinander durch den Katalysator und dann durch den Wärmetauscher gefördert wird.

Ein derartiges Verfahren ist aus der FR-A-2 293 238 bekannt und wird insbesondere für Hydrierreaktionen eingesetzt. Der Wärmetauscher dient hier ausschließlich zur Aufrechterhaltung der Reaktionstemperatur. Da in dem bekannten Verfahren gasförmige Reaktionspartner eingesetzt werden, ist deren Abtrennung aus dem im Kreislauf gefahrenen Reaktionsgemisch unerwünscht.

In anderen bekannten diskontinuierlichen Verfahren zum Führen von heterogen katalysierten Reaktionen werden feste Katalysatoren bei der direkten Zugabe in den Reaktor durch Rührorgane zerkleinert und müssen nach der Reaktion filtriert werden. Dabei ergeben sich häufig erhebliche Verluste an Katalysator und an Produkt. Ein weiteres Problem stellt sich, wenn man die in den älteren deutschen Patentanmeldungen DE-A-38 13 612 und DE-A-38 26 320 genannten Maßnahmen ergreifen will, um eine Chargen- und gegebenenfalls Reaktionszeitverkürzung zu erhalten. In diesem Fall treten Probleme beim Umwälzen des feststoffbeladenen Reaktionsgemisches, insbesondere am Flüssigkeitsverteiler des Filmverdampfers auf.

Der vorliegenden Erfindung liegt die Aufgabe zugrunde, im Verfahren der eingangs genannten Art zum einen die Verluste an Katalysator und an Produkt zu vermeiden und zum anderen das Reaktionsgleichgewicht während des Reaktionsablaufes zu verschieben bzw. den Reaktionsablauf zu beschleunigen, wodurch einerseits die Produktausbeute erhöht und andererseits die Bildung von Nebenprodukten verringert wird.

Diese Aufgabe wird erfindungsgemäß bei einem Verfahren der eingangs genannten Art dadurch gelöst, daß als Wärmetauscher ein Filmverdampfer, insbesondere ein Fallfilm- oder Dünnschichtverdampfer, vorgesehen ist und daß in dem Filmverdampfer die leichter flüchtigen Reaktionsprodukte aus dem Reaktionsgemisch abgetrennt werden.

Im erfindungsgemäßen Verfahren strömt das Reaktionsgemisch nach Durchlaufen des Festbettkatalysators durch den Verdampfer, wobei leichter flüchtige Komponenten verdampft werden, so daß das Reaktionsgemisch auf die Produktseite hin verschoben wird. So kann z. B. bei einer Veresterungsreaktion das entstehende Reaktionswasser direkt nach dem Katalysator, d. h. direkt nach dem Entstehen abgetrennt werden.

Wenn die Reaktion eine von Ionenaustauschern mit Sulfonsäuregruppen katalysierte Veresterungsreaktion ist, wird so die mögliche Hydrolyse der Sulfonsäure, d. h. die Abspaltung der katalytisch aktiven sauren Gruppen reduziert bzw. verhindert.

Grundsätzlich kommen für diese Erfindung jedoch sämtliche festen Katalysatoren in Betracht. So können als Katalysatoren basische oder saure Anionen- oder Kationenaustauscher auf organischer oder anorganischer Basis oder sauer eingestellte Tonerden bzw. Zeolith oder speziell aufbereitete Bleicherden eingesetzt werden.

Das im Festbettkatalysator vorgelegte grobkörnige Katalysatormaterial wird durch geeignete Elemente, z. B. durch Spaltsiebe, zurückgehalten und gelangt nicht in den Rührkessel. So kann dieses Katalysatormaterial für nachfolgende Chargen wieder eingesetzt werden. Das Abfiltrieren eines festen bzw. das Auswaschen eines homogenen Katalysators entfällt somit.

Um die Abtrennwirkung des entstandenen leichter flüchtigen Reaktionsproduktes zu verstärken, wird der Filmverdampfer bevorzugt mit Unterdruck betrieben.

Ferner kann es vorteilhaft sein, wenn während der Herstellung der Druck im Filmverdampfer abgesenkt wird, insbesondere beginnend bei Normaldruck. So kann das Reaktionsgleichgewicht entsprechend dem Fortschreiten der Reaktion in einer gewünschten Lage gehalten werden.

Wenn die Trennung von Edukten und Nebenprodukten nicht durch einfache Destillation möglich ist, wird vorgeschlagen, daß zusätzlich eine an den Reaktor angeschlossene Rektifikationskolonne verwendet wird, an der die leichter flüchtigen Reaktionsprodukte abgetrennt werden, um so eine bessere Trennwirkung als die mit einem Filmverdampfer allein erzielte zu erreichen. Ohne diese Maßnahme wird nämlich oft nicht nur das Reaktionsprodukt, sondern auch mindestens eine Komponente des Ausgangsproduktes mitabgetrennt, so daß die entsprechende Menge des Ausgangsproduktes nachdosiert werden muß. Das verstärkte Nachdosieren läßt sich also vermeiden, wenn die leichter flüchtigen Komponenten des Reaktionsgemisches vor dem Abtrennen im Filmverdampfer rektifiziert werden. Dieses Verfahren findet z. B. Anwendung bei Veresterungen, bei denen ein Edukt, z. B. ein kurzkettiger Alkohol, von einem Nebenprodukt, z. B. Wasser, durch eine Rektifikation getrennt wird, um das Nachdosieren des Alkohols zu vermeiden.

Falls dennoch Ausgangsprodukte im Filmverdampfer mitabgetrennt werden, insbesondere bei Verzicht auf die genannte Rektifikation, wird vorgeschlagen, daß die beim Abtrennen der leichter flüchtigen Reaktionsprodukte mitabgetrennten Ausgangsprodukten nachdosiert werden, um eine Verschiebung des Reaktionsgleichgewichtes zu verhindern.

Der Einsatz des erfindungsgemäßen Verfahrens für Veresterungs- und/oder Umesterungsreaktionen ist vorteilhaft, insbesondere für die Herstellung von Wachsestern oder Riechstoffen. Besonders vorteilhaft ist es, wenn das erfindungsgemäße Verfahren für Umacetalisierungs- und/oder Acetalbildungsreaktionen eingesetzt wird, vor allem zur Herstellung von Formaldehydethyl-Cyclododecylacetal. Bei dieser Substanz handelt es sich um den warenzeichenrechtlich geschützten Stoff Boisambrene Forte.

Bei Reaktionen, bei denen ein druckabhängiges Azeotrop zwischen einem Edukt und einem Reaktionsprodukt auftritt, ist es vorteilhaft, die Reaktion bei dem Druck durchzuführen, bei dem eine maximale Rückführung des Eduktes gewährleistet werden kann. Insbesondere bei der Herstellung von Boisambrene Forte (R) wird ferner vorgeschlagen, daß die Reaktion bei erhöhtem Druck bis zu 6 bar absolut, insbesondere bis zu 4 bar absolut durchgeführt wird. Die Druckerhöhung führt hier zu einer Reduzierung der nachzudosierenden Ethylalmenge aufgrund der Druckabhängigkeit des Azeotrops Ethylal/Ethanol.

Die Erfindung betrifft auch eine Anlage zum diskontinuierlichen heterogen katalysierten Herstellen von thermisch empfindlichen Produkten bei erhöhten Temperaturen mit einem Reaktor, einem außerhalb des Reaktors angeordneten, an diesen angeschlossenen Wärmetauscher, einem vorgeschalteten Katalysatorbehälter mit einem Festbettkatalysator und einer Pumpe zum kontinuierlichen Fördern des Reaktionsgemisches im Kreislauf nacheinander durch den Katalysator und dann durch den Wärmetauscher.

Eine derartige Anlage ist ebenfalls aus der schon genannten FR-A-2 293 238 bekannt.

Zur Lösung der oben genannten Aufgabe wird vorgeschlagen, daß als Wärmetauscher ein Filmverdampfer, insbesondere ein Fallfilmverdampfer oder Dünnschichtverdampfer vorgesehen ist, der zum Abtrennen der leichter flüchtigen Reaktionsprodukte unter Vakuum betreibbar ist.

Es wird auch vorgeschlagen, daß der Katalysatorbehälter Elemente zum Zurückhalten von Katalysatormaterial aufweist.

Weitere, oben genannte Vorteile ergeben sich, wenn die Anlage zum Durchführen der Reaktion bei erhöhtem Druck ausgelegt ist.

Im folgenden wird die Erfindung anhand von Ausführungsbeispielen unter Bezugnahme auf die Zeichnungen näher erläutert. Es zeigen:
- Figur 1:: ein erstes Ausführungsbeispiel mit einem Dünnschichtverdampfer und einem außen liegenden Katalysatorfestbett und
- Figur 2:: ein zweites Ausführungsbeispiel der erfindungsgemäßen Anlage mit außenliegendem Katalysatorfestbett Fallfilmverdampfer und Rektifikationskolonne, wobei die letzteren auf den Reaktor aufgesetzt sind.

### Beispiele

### Beispiel 1:

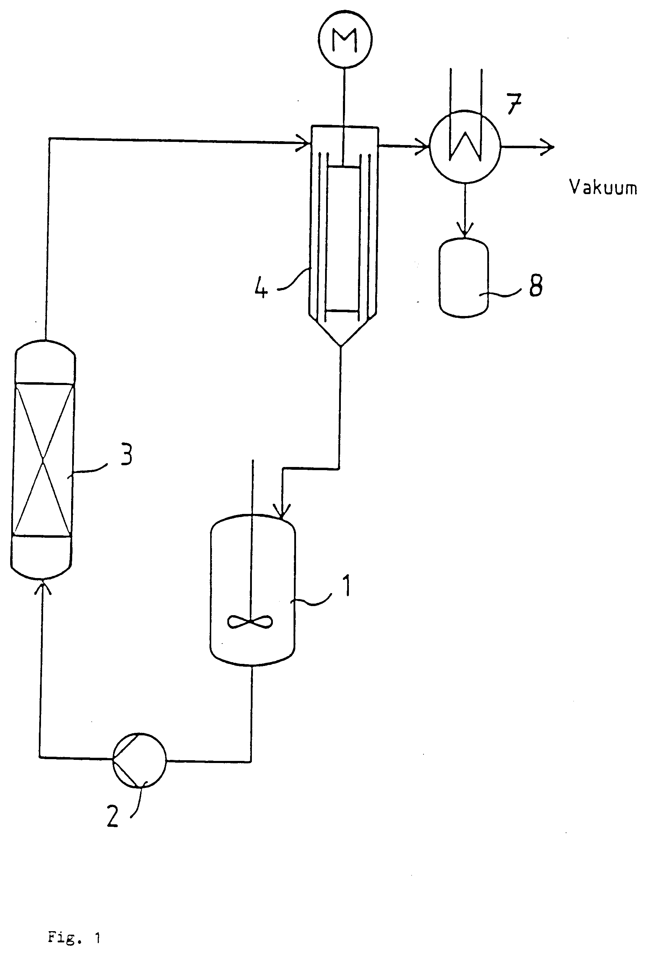

Herstellung eines Wachsesters aus Hexadecansäure und Hexadecylalkohol in der Anlage nach Figur 1.

In einem beheizten Rührbehalter (1) werden 739,5 g Hexadecansäure (2,88 mol) und 706,4 g Fettalkohol (2,91 mol) vorgelegt. Eine Laborkolbenpumpe (2) fördert das Gemisch durch einen beheizten Glasbehälter (3), dem außenliegenden Festbett, in dem 157 g des stark sauren Ionenaustauscherharzes zur Katalyse Amberlite XE 365 (Fa. Rohm und Haas) bei 130 °C vorgelegt wurden, zum Dünnschichtverdampfer (4), in dem das Reaktionswasser destillativ entfernt wird. Während des Versuchs wird stufenweise der Druck von Normaldruck auf 20 mbar abgesenkt. Der Reaktionsfortschritt wird über den Säuregehalt (SZ) im Reaktionsgemisch überwacht. Nach 5 Stunden wird ein Umsatz von 98,3 % bezogen auf die Säure erreicht. Im Destillatbehälter (8) wird eine Destillatmenge von 43 g H₂0 aufgefangen, die restliche Wassermenge befindet sich in der Kühlfalle (7) vor der Vakuumpumpe.

### Beispiel 2:

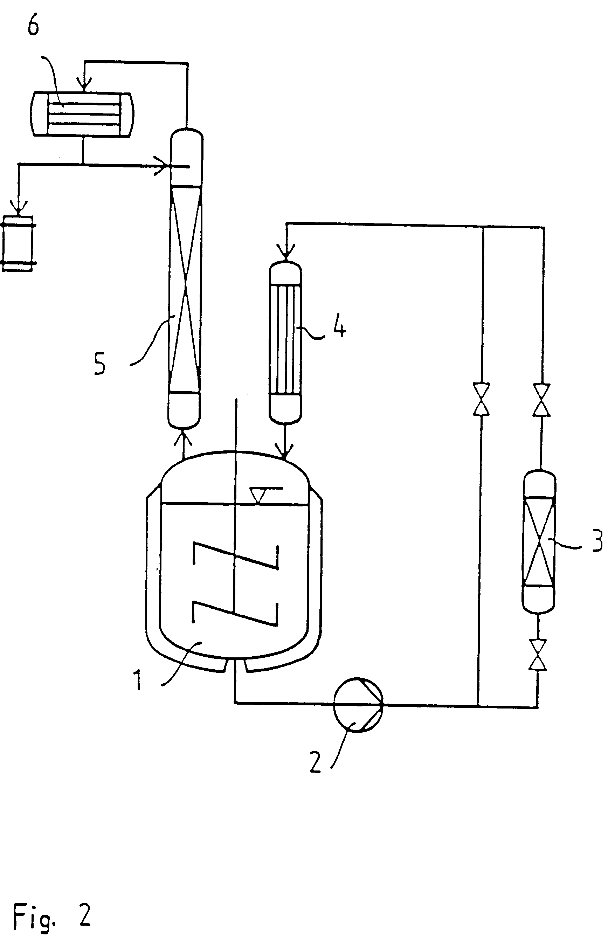

Herstellung von Formaldehyd-ethyl-cyclododecylacetal (Boisambrene Forte^{(R)} (Figur 2)

Formaldehyd-ethyl-cyclododecylacetal ist ein Riechstoff, dessen erstes Herstellverfahren in der deutschen Patentschrift 24 27 500, ein verbessertes in den Patentanmeldungen DE-OS 30 30 543 und DE-0S 30 30 590 offenbart ist.

Das bisherige Verfahren sieht zur Katalyse das Zugeben von konzentrierten flüssigen Säuren oder das Einrühren von festen Katalysatoren vor.

Im erfindungsgemäßen Verfahren werden die Reaktanden Cyclododecanol und Ethylal im Molverhältnis 1:3 in einen beheizbaren Rührkessel (1) vorgelegt. Eine Umwälzpumpe (2) fördert das Reaktionsgemisch durch einen außenliegenden Katalysatorbehälter (3), welcher begleitbeheizt ist und das feste Katalysatormaterial enthält. Als Katalysatormaterial werden entsprechend den bekannten Verfahren entweder sauer eingestellte Tonmineralien (z.B. KSF der Firma Südchemie) oder saure Kationenaustauscher auf organischer oder anorganischer Basis benutzt. Abweichend von den genannten Patentschriften wird jedoch grobkörniges Material mit einem Partikeldurchmesser größer als 0,1 mm eingesetzt.

Das Reaktionsgemisch strömt anschließend durch einen Fallfilmverdampfer (4), worin die leichter flüchtigen Komponenten (Ethanol, Ethylal) teilweise verdampft werden. Die Dämpfe werden in der dem Reaktor aufgesetzten Rektifikationskolonne (5) bis zum Azeotrop aufkonzentriert (ca. 40 Massen % Ethanol), im Rücklaufkondensator (6) kondensiert und teilweise als Destillat abgezogen. Die mitentzogene Menge an Ethylal (chemische Bezeichnung: Diethoxymethan oder Formaldehyddiethylacetal) wird nachdosiert.

Die Reaktion wird bei Siedetemperatur durchgeführt, nämlich anfänglich bei Normaldruck bei ca. 90 °C, gegen Ende der Reaktion bei ca. 110 °C.

Nach etwa 10 - 14 Stunden Reaktionszeit erhält man etwa 99 % Umsatz, bezogen auf Cyclododecanol, bei einer Ausbeute von 80 - 85 % am Wertprodukt Ethylcyclododecylformal.

Im einzelnen wird Boisambrene Forte ^{(R)} erfindungsgemäß folgendermaßen hergestellt:

140 g Cyclododecanol und 238,4 Ethylal (Diethoxymethan) werden in einem beheizten Laborrührkessel (1) (vergleiche Figur 2) vorgelegt. Im Katalysatorbehälter (3) befinden sich 7 g eines Montmorillonit-Katalysators der Firma Südchemie, mit der Bezeichnung KSF/0 Granulat.

Das Reaktionsgemisch wird auf 75 °C aufgeheizt und danach mit Hilfe der Laborpumpe (2) über den Katalysatorbehälter (3) umgewälzt. Die Temperatur der Rührkessel- und Fallfilmverdampfer-Beheizung wird langsam erhöht, und bei einer Reaktionsgemischtemperatur von ca. 90 °C fällt das erste Destillat über Kopf der Kolonne (5) an. Das Rücklaufverhältnis am Kolonnenkopf wird auf 2 eingestellt (1 s Abnahme: 2 s Rücklauf). Immer wenn 15 ml Destillat angefallen sind, werden 10 ml reines Ethylal nachdosiert.

In knapp 12 Stunden werden damit insgesamt 90 ml Destillat (Ethanol/Ethylalgemisch) dem Reaktionssystem entzogen und durch 60 ml reines Ethylal ersetzt. Das angefallene Destillat enthält laut gaschromatographischer Analyse etwa 37 % Ethanol.

Das dabei entstandene Rohprodukt wird vom restlichen Ethylal/Ethanol durch Abdestillieren befreit und enthält laut gaschromatographischer Analyse 83,8 % Boisambrene Forte und 1,1 % nicht umgesetztes Cyclododecanol. Die restlichen 15,1 % bestehen im wesentlichen aus Dicyclododecylformal sowie Spuren von Nebenprodukten.

### Bezugszeichenliste

- 1: Reaktor
- 2: Umwälzpumpe
- 3: Katalysatorbehälter
- 4: Wärmeübertrager/Fallfilmverdampfer (Fig. 2) bzw. Dünnschichtverdampfer (Fig. 1)
- 5: Rektifikationskolonne
- 6: Rücklaufkondensator
- 7: Kondensator (Kühlfalle)
- 8: Destillatbehälter

## Patentansprüche

1. Diskontinuierliches Verfahren zum Führen einer heterogen katalysierten und bei erhöhter Temperatur ablaufenden Reaktion, bei der thermisch empfindliche Produkte entstehen, wobei zum Energieeintrag ein vom Reaktor (1) unterschiedlicher Wärmetauscher und als Katalysator ein Festbettkatalysator (3) eingesetzt wird und das Reaktionsgemisch kontinuierlich im Kreislauf nacheinander durch den Katalysator (3) und dann durch den Wärmetauscher (4) gefördert wird,
**dadurch gekennzeichnet**,
daß als Wärmetauscher ein Filmverdampfer (4), insbesondere ein Fallfilm- oder Dünnschichtverdampfer, vorgesehen ist und daß in dem Filmverdampfer die leichter flüchtigen Reaktionsprodukte aus dem Reaktionsgemisch abgetrennt werden.

2. Verfahren nach Anspruch 1,
dadurch gekennzeichnet,
daß zusätzlich eine an den Reaktor (1) angeschlossene Rektifikationskolonne (5) verwendet wird, an der die leichter flüchtigen Reaktionsprodukte abgetrennt werden, wenn die Trennung von Edukten und Nebenprodukten nicht durch einfache Destillation möglich ist.

3. Verfahren nach Anspruch 1 oder 2,
dadurch gekennzeichnet,
daß die beim Abtrennen der leichter flüchtigen Reaktionsprodukte mitabgetrennten Ausgangsprodukte nachdosiert werden.

4. Verfahren nach einem der Ansprüche 1 bis 3,
dadurch gekennzeichnet,
daß es für Veresterungs- und/oder Umesterungsreaktionen eingesetzt wird.

5. Verfahren nach einem der Ansprüche 1 bis 4,
dadurch gekennzeichnet,
daß es für Umacetalisierungs- und/oder Acetalbildungsreaktionen eingesetzt wird, insbesondere daß Formaldehyd-ethylcyclododecylacetal hergestellt wird.

6. Verfahren nach einem der Ansprüche 1 bis 5 zum Herstellen von Formaldehyd-ethylcyclododecylacetal,
dadurch gekennzeichnet,
daß die Reaktion bei erhöhtem Druck bis zu 6 bar absolut, insbesondere bis zu 4 bar absolut durchgeführt wird.

7. Anlage zum diskontinuierlichen heterogen katalysierten Herstellen von thermisch empfindlichen Produkten bei erhöhten Temperaturen mit einem Reaktor (1), einem außerhalb des Reaktors (1) angeordneten, an diesen angeschlossenen Wärmetauscher (4), einem vorgeschalteten Katalysatorbehälter (3) mit einem Festbettkatalysator und einer Pumpe (2) zum kontinuierlichen Fördern des Reaktionsgemisches im Kreislauf nacheinander durch den Katalysator (3) und dann durch den Wärmetauscher (4),
**dadurch gekennzeichnet**,
daß als Wärmetauscher ein Filmverdampfer (4), insbesondere ein Fallfilm- oder Dünnschichtverdampfer vorgesehen ist, der zum Abtrennen der leichter flüchtigen Reaktionsprodukte unter Vakuum betreibbar ist.

8. Anlage nach Anspruch 7,
dadurch gekennzeichnet,
daß der Katalysatorbehälter (3) Elemente zum Zurückhalten von Katalysatormaterial aufweist.

9. Anlage nach Anspruch 7 oder 8,
dadurch gekennzeichnet,
daß sie zum Durchführen der Reaktion bei erhöhtem Druck ausgelegt ist.

## Claims

1. A discontinuous process for conducting a heterogeneously catalyzed reaction taking place at elevated temperature, in which heat-sensitive products are formed, a heat exchanger (4) different from the reactor (1) being used for heating and a fixed bed catalyst (3) being used as the catalyst and the reaction mixture being continuously circulated in succession through the catalyst (3) and then through the heat exchanger (4), characterized in that the heat exchanger (4) is a film evaporator, more especially a falling film or thin layer evaporator, and in that the more readily volatile reaction products are separated from the reaction mixture in the film evaporator.

2. A process as claimed in claim 1, characterized in that a rectification column (5) connected to the reactor (1) in which the more readily volatile reaction products are removed, is additionally used where educts and secondary products cannot be separated simply by distillation.

3. A process as claimed in claim 1 or 2, characterized in that the starting products separated with the more volatile reaction products are replenished.

4. A process as claimed in any of claims 1 to 3, characterized in that it is used for esterification and/or transesterification reactions.

5. A process as claimed in any of claims 1 to 4, characterized in that it is used for transacetalization and/or acetal-forming reactions, more especially for the production of formaldehyde ethyl cyclododecyl acetal.

6. A process as claimed in any of claims 1 to 5 for the production of formaldehyde ethyl cyclododecyl acetal, characterized in that the reaction is carried out under an elevated pressure of up to 6 bar absolute, more particularly up to 4 bar absolute.

7. A plant for the discontinuous heterogeneously catalyzed production of heat-sensitive products at elevated temperature comprising a reactor (1), a heat exchanger (4) arranged outside and connected to the reactor (1), a catalyst container (3) containing a fixed-bed catalyst preceding the heat exchanger and a pump (2) for continuously circulating the reaction mixture in succession through the catalyst (3) and then through the heat exchanger (4), characterized in that the heat exchanger (4) is a film evaporator, more especially a falling film evaporator or thin layer evaporator, which is designed to separate the more readily volatile reaction products in vacuo.

8. A plant as claimed in claim 7, characterized in that the catalyst container (3) comprises elements for retaining catalyst material.

9. A plant as claimed in claim 7 or 8, characterized in that it is designed to carry out the reaction under elevated pressure.

## Revendications

1. Procédé en discontinu pour la conduite d'une réaction qui se déroule à température élevée et en catalyse hétérogène, dans laquelle des produits sensibles à la chaleur se forment, procédé dans lequel pour l'introduction d'énergie un échangeur de chaleur distinct du réacteur (1) et comme catalyseur, un catalyseur à lit solide (3) sont mis en oeuvre et le mélange réactionnel est transporté en continu en circuit l'un après l'autre à travers le catalyseur (3) et ensuite à travers l'échangeur de chaleur (4),
caractérisé en ce que comme échangeur de chaleur, un évaporateur pelliculaire (4), en particulier un évaporateur à pellicule tombante ou un évaporateur en couche mince, est prévu et en ce que dans l'évaporateur pelliculaire les produits de réaction plus facilement volatils sont séparés du mélange réactionnel.

2. Procédé selon la revendication 1, caractérisé en ce qu'en supplément une colonne de rectification (5) branchée sur le réacteur (1) est utilisée, sur laquelle les produits de réaction plus facilement volatils sont chassés, lorsque la séparation des produits de départ et des sous-produits n'est pas possible par une simple distillation.

3. Procédé selon la revendication 1 ou 2, caractérisé en ce que lors de l'évacuation des produits de réaction plus facilement volatils, les produits de départ évacués en même temps, sont ajoutés finalement par portions.

4. Procédé selon l'une des revendications 1 à 3, caractérisé en ce qu'il est mis en oeuvre pour des réactions d'estérification et/ou de transestérification.

5. Procédé selon l'une des revendications 1 à 4, caractérisé en ce qu'il est mis en oeuvre pour des réactions de transacétalisation et/ou de formation d'acétal, en particulier en ce que le formaldéhyde-éthyl-cyclododécylacétal est produit.

6. Procédé selon l'une des revendications 1 à 5 pour la production de formaldéhyde-éthyl cyclododécylacétal, caractérisé en ce que la réaction est effectuée à pression élevée allant jusqu'à 6 bar absolus, en particulier jusqu'à 4 bar absolus.

7. Installation pour la production en discontinu par catalyse hétérogène de produits sensibles à la chaleur, à température élevée avec un réacteur (1), un échangeur de chaleur (4) disposé à l'extérieur du réacteur (1) mais branché sur celui-ci, un récipient pour catalyseur pré-connecté (3) avec un catalyseur en lit solide et une pompe (2) en vue du transport en continu du mélange réactionnel en circuit l'un après l'autre à travers le catalyseur (3) et ensuite à travers l'échangeur de chaleur (4), caractérisée en ce que comme échangeur de chaleur un évaporateur pelliculaire (4), en particulier un évaporateur à film en couche mince, est prévu qui peut fonctionner en vue de l'évacuation des produits de réaction plus facilement volatils sous vide.

8. Installation selon la revendication 7, caractérisée en ce que le récipient pour catalyseur (3) possède des éléments pour retenir le matériau du catalyseur.

9. Installation selon la revendication 7 ou 8, caractérisée en ce qu'elle est étudiée pour l'exécution de la réaction à pression élevée.
